# EUROPEAN PATENT APPLICATION

(11) **EP 2 324 782 A1**
(43) Date of publication of application: **25.05.2011**
(21) Application number: 10251940.2
(22) Date of filing: 17.11.2010
(51) Int. Cl.: A61B 17/34, A61M 39/04

(54) **Port fixation device**

(30) Priority: 18.11.2009 US 262314 P; 21.10.2010 US 908974
(71) Applicant: Tyco Healthcare Group LP, New Haven, CT 06511 (US)
(72) Inventor: Fischvogt, Gregory, Hamden, CT 06514 (US)
(74) Representative: Soames, Candida Jane

(57) **Abstract**

A port fixation device is disclosed. The port fixation device comprises a body portion and a longitudinal slit. The body portion has an elongated tubular shaped and has an interior lumen defining a longitudinal axis therethrough. The interior lumen is configured for reception of a surgical access port. The longitudinal slit is disposed in the body portion between a first longitudinal edge thereof and a second longitudinal edge thereof, and extends between a proximal portion and a distal portion of the body portion. The body portion is configured to expand radially outwardly to accommodate a surgical access port therein.

## Description

### CROSS-REFERENCE TO RELATED APPLICATIONS

This application claims priority to and the benefit of U.S. Provisional Application Serial No. 61/262,314 filed on November 18, 2009, the entire contents of which is incorporated herein by reference.

### BACKGROUND

### Technical Field

The present disclosure relates to a surgical apparatus. More particularly, the present disclosure relates to a surgical portal apparatus for permitting introduction of a portal into a body wall and maintaining the portal in fixed relation during a surgical procedure.

### Background of Related Art

In minimally invasive surgical procedures, including endoscopic and laparoscopic surgeries, a surgical portal permits the introduction of a variety of surgical instruments into a body cavity or opening. A surgical portal is introduced through a cavity or incision to provide access to an underlying surgical site in the body. The incision is typically made using an obturator having a blunt or sharp radius within the passageway of the surgical portal. For example, a trocar has a cannula, a tube of rigid, thin wall construction, through which an obturator may be distally passed. The obturator is utilized to penetrate a body wall, such as an abdominal wall, or to introduce the portal through the body wall, and is then removed to permit introduction of surgical instrumentation through the portal to perform the surgical procedure.

These procedures may present issues with respect to maintenance of the portal within the body wall, particularly, when exposed to a pressurized environment and/or over insertion of the portal and possible impingement of underlying body organs.

### SUMMARY

The present disclosure relates to a port fixation device. The port fixation device comprises a body portion and a longitudinal slit. The body portion has an elongated tubular shaped and has an interior lumen defining a longitudinal axis therethrough. The interior lumen is configured for reception of a surgical access port. The longitudinal slit is disposed in the body portion between a first longitudinal edge thereof and a second longitudinal edge thereof, and extends between a proximal portion and a distal portion of the body portion. The body portion is configured to expand radially outwardly to accommodate a surgical access port therein.

The present disclosure also relates to a surgical access apparatus comprising a surgical access port and a port fixation device. The port fixation device comprises a body portion having an elongated tubular shape and an interior lumen defining a longitudinal axis therethrough. The interior lumen is configured for reception of the surgical access port. A longitudinal slit is disposed in the body portion between a first longitudinal edge thereof and a second longitudinal edge thereof, and extends between a proximal portion and a distal portion of the body portion. The body portion is configured to expand radially outwardly to accommodate the surgical access port at least partially therein.

### BRIEF DESCRIPTION OF THE DRAWINGS

Embodiments of the presently disclosed port fixation device and surgical access port are described herein with reference to the accompanying drawings, wherein:

FIG. 1 is a perspective view of a surgical access port for use with port fixation devices of the present disclosure;

FIG. 2 is a perspective view of the surgical access port of FIG. 1 and a port fixation device in an initial, contracted position inserted into a patient's tissue according to an embodiment of the present disclosure;

FIG. 3 is a perspective view of the surgical access port of FIGS. 1 and 2 inserted into the port fixation device of FIG. 2 in a second, expanded position and within tissue;

FIG. 4 is a perspective view of a port fixation device inserted into a patient's tissue in accordance with another embodiment of the present disclosure and shown in an initial, contracted position;

FIG. 5 is a perspective view of a surgical access port inserted into the port fixation device of FIG. 4 in a second, expanded position and within tissue; and

FIG. 6 is a perspective view of a port fixation device in accordance with another embodiment of the present disclosure.

Other features of the present disclosure will become apparent from the following detailed description, taken in conjunction with the accompanying drawings, which illustrate, by way of example, various principles of the present disclosure.

### DETAILED DESCRIPTION OF THE EMBODIMENTS

Particular embodiments of the present disclosure will be described herein with reference to the accompanying drawings. As shown in the drawings and as described throughout the following description, and as is traditional when referring to relative positioning on an object, the term "proximal" refers to the end of the apparatus that is closer to the user and the term "distal" refers to the end of the apparatus that is farther from the user. In the following description, well-known functions or constructions are not described in detail to avoid obscuring the present disclosure in unnecessary detail.

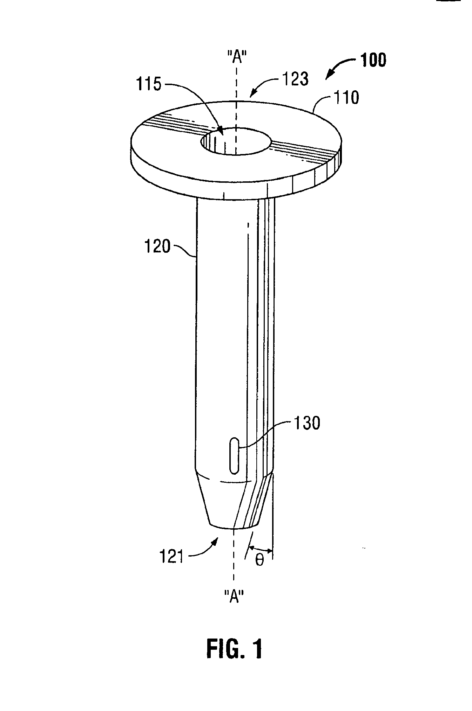

Referring now to FIG. 1 of the drawings, a surgical access port 100 is dimensioned for insertion within tissue to access an underlying tissue site. Access port 100 includes a flange 110 and an elongate member 120 having an interior passage 115 that defines a longitudinal axis "A-A." The interior passage 115 is dimensioned for reception of surgical objects (not shown).

The surgical access port 100 has a proximal end 123 and a distal end 121. The distal end 121 may be tapered at an angle 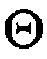 . An instrument-viewing window 130 is illustrated and is proximally disposed with respect from the distal end 121. The window 130 is an aperture that extends from the interior passage 115 through the port wall of the elongate member 120.

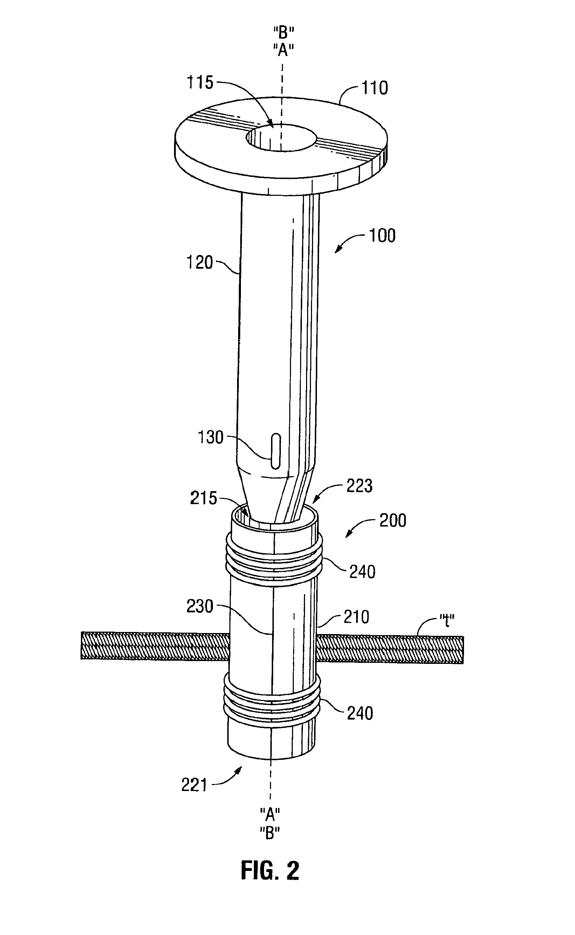

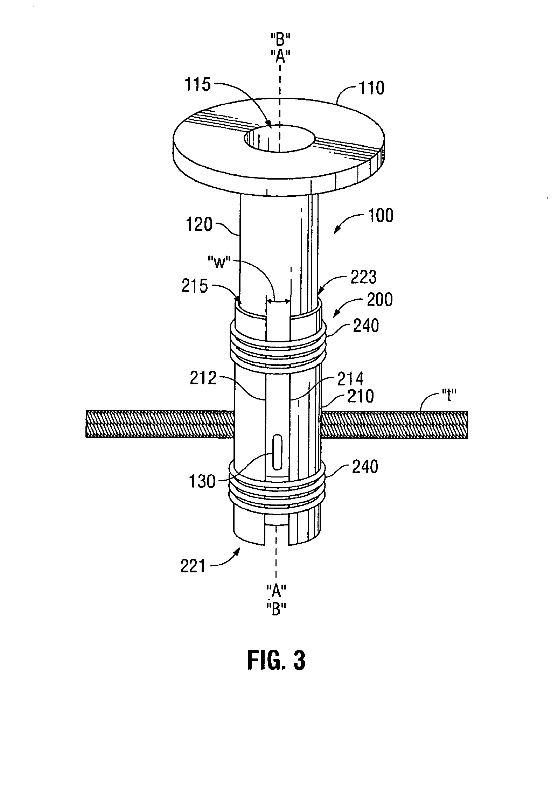

Referring now to FIGS. 2 and 3, a port fixation device 200 is illustrated in connection with access port 100 of FIG. 1. Port fixation device 200 includes a body portion 210 having an elongate tubular shape and an interior lumen 215 that defines a longitudinal axis "B-B." Body portion 210 includes a slit 230 extending between a distal end 221 and a proximal end 223. Slit 230 is defined between a first longitudinal edge 212 and a second longitudinal edge 214 of body portion 214 (FIG. 3). Port fixation device 200 is configured to move between an initial, contracted position (FIG. 2) and a second, expanded position (FIG. 3). A plurality of elastomeric members 240 surround the body portion 210 at various locations along the length of body portion 210 and are configured to bias port fixation device 200 towards its initial, contracted position. Elastomeric members 240 are configured to exert an inwardly directed force against body portion 210.

It is envisioned that body portion 210 includes grooves (not shown) that circumferentially surround body portion 210, such that each groove is configured and dimensioned to accept at least part of an elastomeric member 240 therein. Interior lumen 215 of body portion 210 is configured to accept a portion of tapered distal end 121 of the access port 100.

In use, as illustrated in FIG. 3, the body portion 210 is inserted into an incision in the tissue of a patient and then the surgical access port 100 is generally co-axially placed within the body portion 210. (As shown in FIG. 3, axes "A-A" and "B-B" are coaxial.) The external circumference of the non-tapered portion of elongate member 120 is larger than the internal circumference of the body portion 210. Thus, as surgical access port 100 passes through body portion 210, body portion 210 expands radially outwardly against the force supplied by elastomeric members 240 acting thereagainst. As a result, first longitudinal edge 212 and second longitudinal edge 214 of slit 230 are forced apart, thus expanding a width "w" of slit 230 (FIG. 3).

The outward force from the insertion of surgical access port 100 causes both the elastomeric members 240 and the incision in the patient to radially expand. The incision exerts a resulting inwardly directed force against body portion 210, thus helping secure port fixation device 200 within a patient's incision. Additionally, since elastomeric members 240 exert an inwardly directed force, body portion 210 is compressed against elongate member 120 of surgical access port 100. As a result, surgical access port 100 is removably secured within a patient's incision.

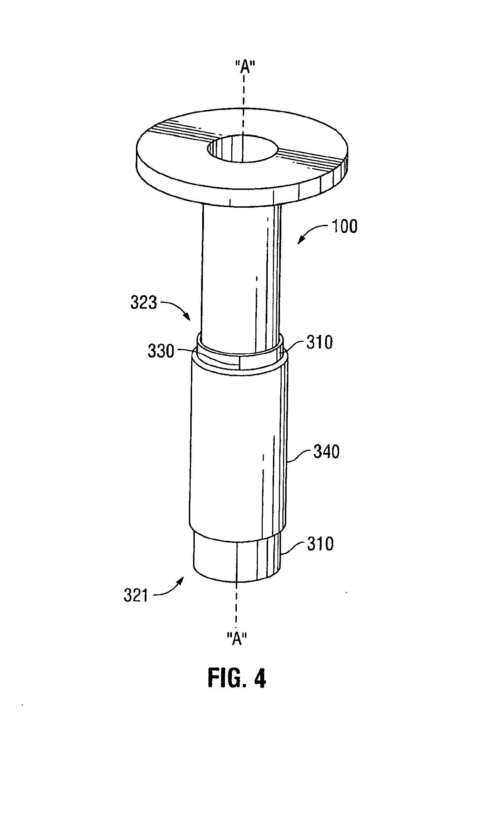

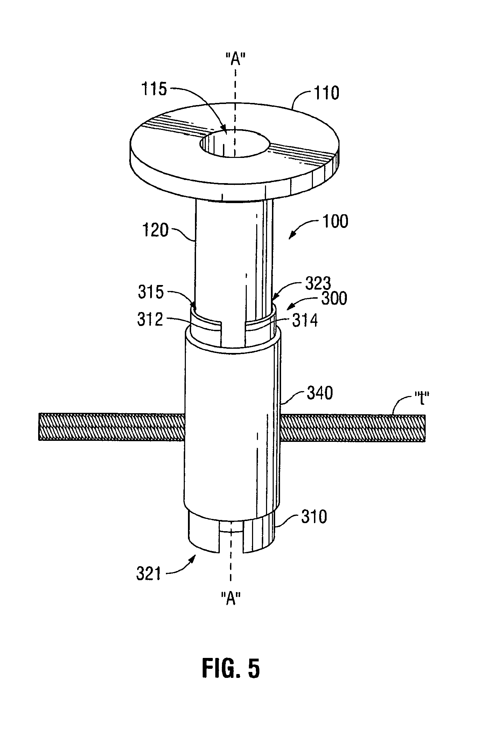

Another embodiment of a port fixation device 300 is shown in FIGS. 4 and 5. Port fixation device 300 is substantially similar to port fixation device 200. In particular, port fixation device 300 includes a body portion 310 including a slit 330 extending between a distal end 321 and a proximal end 323. Slit 330 is defined between a first longitudinal edge 312 and a second longitudinal edge 314 of body portion 310 (FIG. 5). Additionally, port fixation device 300 includes a sleeve-like elastomeric member 340. As shown in FIGS. 4 and 5, elastomeric member 340 extends a majority of the length of body portion 310. FIG. 4 illustrates elastomeric member 340 in an initial, contracted position. FIG. 5 illustrates elastomeric member 340 is a second, expanded position with surgical access port 100 partially inserted therethrough. Additionally, while not explicitly illustrated, body portion 310 may include a groove therein for at least partially accepting at least a portion of sleeve-like elastomeric member 340 therein.

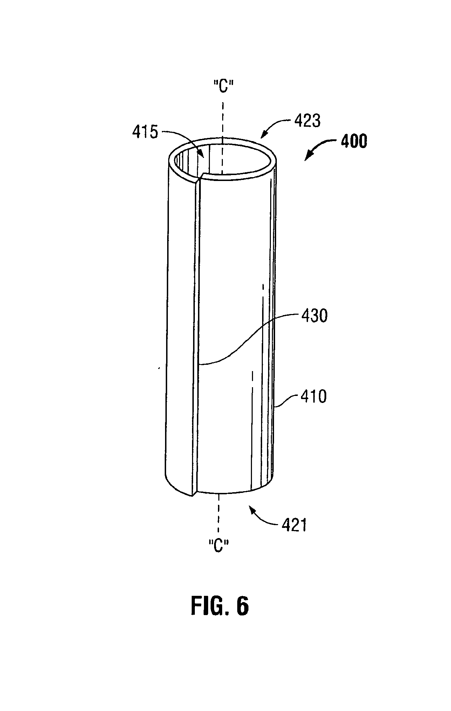

Another embodiment of a port fixation device 400 is shown in FIG. 6. Port fixation device 400 is made from an elastomeric material and includes an interior lumen 415 defining a longitudinal axis "C-C" and a slit 430 extending between a distal end 421 and a proximal end 423. The interior lumen 415 is sized to partially accept the distal end 121 of the access port 100. It is envisioned that making port fixation device 400 of an elastomeric material alleviates the need for additional elastomeric members, however it is envisioned that additional elastomeric members may be used in combination with port fixation device 400.

While in each embodiment disclosed herein, the slits 230, 330, 430 are disclosed as being linear, it is envisioned and within the scope of the present disclosure that at least a portion of slit 230, 330 or 430 is non-linear, e.g. curved.

It will be understood that various modifications may be made to the embodiments of the presently disclosed surgical portal devices. Therefore, the above description should not be construed as limiting, but merely as exemplifications of embodiments. Those skilled in the art will envision other modifications within the scope and spirit of the present disclosure.

## Claims

1. A port fixation device, comprising:
a body portion having an elongated tubular shape and an interior lumen defining a longitudinal axis therethrough, the interior lumen configured for reception of a surgical access port; and
a longitudinal slit disposed in the body portion between a first longitudinal edge thereof and a second longitudinal edge thereof, the longitudinal slit extending between a proximal portion and a distal portion of the body portion, the body portion being configured and dimensioned to expand radially outwardly to accommodate a surgical access port therein.

2. The port fixation device of Claim 1, wherein the longitudinal slit extends the entire length between a proximal end and a distal end of the body portion.

3. The port fixation device of Claim 1 or Claim 2, further comprising at least one elastomeric member surrounding a periphery of the body portion.

4. The port fixation device of Claim 3, wherein the elastomeric member is configured to bias the body portion towards an initial, contracted position where the first longitudinal edge of the body portion is in substantial contact with the second longitudinal edge of the body portion.

5. The port fixation device of Claim 4, wherein insertion of a surgical access port is configured to move the body portion towards a second, expanded position where the first longitudinal edge of the body portion is spaced from the second longitudinal edge of the body portion.

6. The port fixation device of Claim 4 or Claim 5, wherein the at least one elastomeric member is a plurality of ring-like elastomeric members.

7. The port fixation device of Claim 4 or Claim 5, wherein the at least one elastomeric member is a single, sleeve-like elastomeric member.

8. The port fixation device of Claim 7, wherein the sleeve-like elastomeric member extends substantially the length of the body wall between a proximal end and a distal end.

9. The port fixation device of any preceding Claim, wherein the body portion is made from an elastomeric material.

10. A surgical access apparatus, comprising:
a surgical access port including a elongated portion; and
a port fixation device, comprising:
a body portion having an elongated tubular shape and an interior lumen defining a longitudinal axis therethrough, the interior lumen configured for reception of the surgical access port; and
a longitudinal slit disposed in the body portion between a first longitudinal edge thereof and a second longitudinal edge thereof, the longitudinal slit extending between a proximal portion and a distal portion of the body portion;
wherein the body portion is configured to expand radially outwardly to accommodate the surgical access port at least partially therein.

11. The surgical access apparatus of Claim 10, wherein a distal end of the surgical access port is tapered.

12. The surgical access apparatus of Claim 10 or Claim 11, wherein a diameter of the tapered portion of the surgical access port is smaller than a diameter of the body portion of the port fixation device in an initial, contracted position.

13. The surgical access apparatus of Claim 12, wherein a diameter of the elongated portion of the surgical access port is larger than a diameter of the body portion of the port fixation device in an initial, contracted position.

14. The surgical access apparatus of any of Claims 10 to 13, further comprising at least one elastomeric member surrounding a periphery of the body portion of the port fixation device, wherein the elastomeric member is configured to bias the body portion towards an initial, contracted position where the first longitudinal edge of the body portion is in substantial contact with the second longitudinal edge of the body portion.

15. The surgical access apparatus of Claim 14, wherein insertion of the surgical access port is configured to move the body portion towards a second, expanded position where the first longitudinal edge of the body portion is spaced from the second longitudinal edge of the body portion.

16. The surgical access apparatus of Claim 14 or Claim 15, wherein the at least one elastomeric member is a plurality of ring-like elastomeric members.

17. The surgical access apparatus of Claim 14 or Claim 15, wherein the at least one elastomeric member is a single, sleeve-like elastomeric member.
